# FASCICULE DE BREVET EUROPEEN

(11) **EP 1 754 792 B1**
(45) Date de publication et mention de la délivrance du brevet: **30.03.2011**
(21) Numéro de dépôt: 06022204.9
(22) Date de dépôt: 02.04.1999
(51) Int. Cl.: C12Q 1/34, C12Q 1/04

(54) **Nouveaux substrats chromogènes pour la détection d'hydrolases bactériennes**
Neue chromogene Substrate zum Nachweis von bakteriellen Hydrolasen
Novel chromogenous substrates for detecting bacterial hydrolase

(30) Priorité: 02.04.1998 FR 9804332
(43) Date de publication de la demande: 21.02.2007
(62) Demande divisionnaire de: 99911874.8
(73) Titulaire: bioMérieux, 69280 Marcy L'Etoile (FR)
(72) Inventeur: Armstrong, Lyle, Northumberland NE63 8AE (GB); James, Arthur, Newcastle upon Tyne NE21HR (GB); Monget, Daniel, 01150 Saint Sorlin en Bugey (FR); Orenga, Sylvain, 01160 Neuville sur Ain (FR)
(74) Mandataire: Guerre, Dominique

(56) Documents cités:
- EP-A- 0 053 470
- EP-A- 0 138 530
- DE-A- 4 324 392
- FR-A- 2 708 286
- US-A- 4 588 836
- US-A- 4 603 108
- US-A- 4 637 979
- US-A- 4 681 841
- J F CORBETT: "Benzoquinone Imines. Part VIII. Mechanism and Kinetics of the Reaction of p-Benzoquinone Monoimines with Monohydric Phenols." JOURNAL OF THE CHEMICAL SOCIETY B, 1970, pages 1502-1509, XP002087428

## Description

La présente invention concerne la mise en évidence d'enzymes de type peptidase, par l'utilisation de substrats chromogènes efficaces. La présente publication concerne également un procédé d'identification de micro-organismes à la fois simple et fiable.

*Depuis de très nombreuses années, on utilise des substrats particuliers pour permettre la détermination de la présence ou de l'absence d'activités enzymatiques caractéristiques de bactéries. Par le choix des substrats, selon qu'il y a réaction ou non, il est possible de caractériser la nature d'un genre de bactéries ou de différencier des espèces d'un genre bactérien donné.*

*Les substrats synthétiques d'enzymes sont constitués de deux parties, une première partie spécifique de l'activité enzymatique à révéler, et une seconde partie faisant office de marqueur, ci-après appelée partie marqueur.*

*Ces substrats particuliers peuvent être des substrats fluorescents ou chromogènes. En fait, c'est la seconde partie ou partie marqueur qui est fluorescente ou chromogène, lorsqu'elle n'est pas associée à la première partie.*

*Les substrats fluorescents peuvent être de différentes compositions.*

*Tout d'abord les substrats à base d'umbelliférone ou d'aminocoumarine, et ses dérivés substitués en position 2, qui permettent la libération d'un composé fluorescent de couleur variant du bleu au vert sous lampe à ultraviolets (UV) (λₑₓ= 365 nm).*

*Ensuite les substrats à base de résorufine (et dérivés) où il y a libération d'un composé fluorescent rose sous lumière naturelle (λₑₓ= 530 nm).*

*Enfin les substrats à base de fluorescéïne (et dérivés) qui, après dégradation, libère un composé fluorescent jaune* sous *lumière naturelle (λₑₓ= 485 nm).*

Ces substrats sont peu adaptés à une utilisation dans des milieux gélosés, et sont plus utilisés en milieu liquide.

*Les substrats chromogènes peuvent également être de différentes natures*.

*Premièrement, il y a les substrats à base d'indoxyl et ses dérivés qui, en présence d'oxygène, produisent un précipité variant du bleu au rose.*

Leurs applications sont essentiellement limitées aux osidases, estérases et ne concernent pas la détection d'une activité peptidase. Bien adaptés à une utilisation sur support solide (filtre, gélose, gel d'électrophorèse, ...), ils sont par contre moins bien adaptés à une utilisation en milieu aqueux liquide (formation d'un précipité).

*Deuxièmement, il y a les substrats à base d'hydroxyquinoline ou d'esculétine et leurs dérivés, qui en présence de sels de fer, produisent un précipité brun.*

Encore une fois leurs applications sont limitées aux osidases et estérases. Ils sont adaptés à une utilisation sur support solide, et peu adaptés à une utilisation en milieu aqueux liquide.

*Troisièmement, il existe des substrats à base de nitrophénol et nitroaniline et dérivés, où l'on obtient la formation d'un composé jaune.*

ils permettent de détecter les activités osidases et estérases dans le cas de substrats à base de nitrophénol, et des activités peptidases dans le cas de substrats à base de nitroaniline. Cependant, dans le cas de la détection d'activités peptidases, la nitroaniline libérée est toxique pour les bactéries que l'on souhaite identifier ou caractériser, ce qui peut être préjudiciable pour des analyses en cours ou ultérieures. D'autre part, ils sont peu adaptés à une utilisation sur support solide, et mieux adaptés à une utilisation en milieu liquide. De plus, ils sont peu chromogènes du fait du coefficient d'extinction relativement faible de la couleur (jaune) dont le contraste est peu prononcé dans les milieux biologiques.

*Quatrièmement, il y a les substrats à base de naphtol et naphtylamine et ses dérivés*. *Dans ce cas, la réaction s'effectue en deux temps, le naphtol ou naphtylamine libéré par l'activité enzymatique subit un "azo-coupling" en présence d'un sel de diazonium qui est ajouté au moment de la révélation, conduisant à la formation d'un composé insoluble coloré.*

Ils permettent de détecter les activités osidases et estérases par l'intermédiaire du naphtol, et les activités peptidases par l'intermédiaire de la naphtylamine. La réaction d' "azo-coupling" s'effectue dans un milieu souvent chimiquement agressif, toxique pour les bactéries et rendant l'échantillon inutilisable pour d'autres analyses, de plus les naphtylamines sont cancérigènes.

Pour révéler la naphtylamine et donc une activité peptidase, il est aussi possible d'ajouter en fin de réaction enzymatique du p-diméthylaminocinnamaldehyde en milieu acide, à la place d'un sel de diazonium, avec toujours des inconvénients de toxicité vis-à-vis de l'échantillon analysé.

*La demande de brevet* FR-A-2.708.286 *propose d'utiliser un mélange de substrats chromogènes, chaque chromogène fournit une coloration particulière pour une enzyme spécifique qui est différente de la coloration et de l'enzyme associées à l'autre chromogène. Lorsque les deux colorations et dune les deux enzymes sont présentes, il y a formation d'une* « *tierce coloration* ».

Cette technique n'est toutefois pas satisfaisante car en fonction de la faible concentration de l'une des deux enzymes, il n'est pas possible de détecter cette activité enzymatique qui est alors masquée par la coloration associée à l'enzyme dont la concentration est prépondérante.

*Enfin, les brevets* US-A-4,681,841 *et* US-A-4,588,836 *décrivent une méthode indirecte de détection d'une seule activité enzymatique utilisant un couplage entre un aminobenzène et un dérivé aromatique hydroxylé (par exemple l'alpha-naphtol), couplage qui engendre la formation d'un indicateur chromogène en présence d'oxydase. L'un des deux composés (l'aminobenzène) entre dans la composition du substrat de départ; si l'activité enzymatique recherchée est présente, ce composé sera libéré et pourra réagir avec l'autre composé.*

Néanmoins, la présence d'oxydase dans le milieu réactionnel, pour que la mise en évidence de l'enzyme recherchée soit possible, est une absolue nécessité. Pourtant bien que ces informations dissuadent l'homme du métier de rechercher une solution qui n'utilise pas d'oxydase, la demanderesse, par de nombreux tests effectués dans ses laboratoires, a prouvé qu'il est possible de mettre en évidence l'enzyme recherchée par l'intermédiaire de l'utilisation, par exemple, de l'amino-benzène et de l'α-naphtol, sans l'addition d'oxydase.

Il est donc aisé de comprendre qu'il n'existe pas aujourd'hui de substrat chromogène pour révéler au moins une activité peptidase qui soit particulièrement efficace et intéressant.

*Concernant le procédé et le dispositif d'identification, l'état de la technique es*t *c*onstitué *par un procédé d'identification qui prévoit une manipulati*on *en trois étapes.*
- *prélèvement de la colonie à identifier,*
- *réalisation d'un test d'orientation, et*
- *recherche de colonies semblables à celles observées et réalisation d'un inoculum.*

*Le test d'orientation doit être pratiqué avant l'utilisation d'un système d'identification*

*C'est notamment le cas pour la coloration de Gram qui nécessite une observation microscopique.*

Cependant, cette coloration n'est pas toujours facile à réaliser et surtout à interpréter. D'autre part, le coût engendré par ce test est loin d'être négligeable.

L'un des objectifs de la présente invention est donc de créer le lien entre un milieu de culture et les systèmes d'identification et d'antibiogramme en offrant aux biologistes la possibilité d'effectuer un test simple en une étape permettant à la fois la confirmation du résultat de la coloration de Gram et la préparation de l'inoculum. Ainsi le choix des tests d'identification et d'antibiogramme est fiabilisé.

*La demande de brevet* EP-A-0,122,028 *propose une méthode de colorimétrie permettant de détecter la présence d'au moins une enzyme suspectée d'être présente dans un échantillon biologique. Elle préconise de préparer un matériau absorbant monté sur un support rigide, tel qu'un écouvillon, en absorbant dans ce matériau au moins un substrat spécifique de l'enzyme que l'on souhaite détecter. Préalablement à son utilisation, le matériau absorbant contenant le ou les substrats est séché.*

L'invention permet la mise en évidence colorimétrique d'activités enzymatiques de type , peptidase) à l'aide de substrats synthétiques à base de deux composés, ainsi qu'un procédé nouveau qui peut être appliqué à des milieux réactionnels aussi bien liquides que solides.

Elle, propose également un procédé d'identification de micro-organismes, qui oriente l'identification, et permet la récupération desdits micro-organismes pour permettre d'effectuer un inoculum. Cet inoculum permet d'utiliser les mêmes micro-organismes pour une ou plusieurs autres étapes, tels qu'une identification, un antibiogramme.

Après hydrolyse des substrats, la détection des activités enzymatiques est basée sur la formation d'un complexe coloré à partir du couplage oxydatif des deux composés précédemment cités. Ce couplage oxydatif peut être facilité par un oxydant ajouté au milieu réactionnel ou produit lors d'un processus métabolique au sein de ce milieu, au plus simplement par la présence d'oxygène endogène dans ce même milieu.

Selon une première interprétation, la présente invention concerne une association de deux entités distinctes, l'une étant une molécule non chromogène et l'autre étant le substrat permettant de détecter la présence de l'activité enzymatique d'au moins une enzyme. Le substrat est caractérisé par le fait qu'il est constitué d'une partie spécifique de l'enzyme, et d'une molécule non chromogène différente de la première, qui constitue la partie marqueur du substrat ; les deux molécules non chromogènes réagissent ensemble quand elles sont sous forme libre et peuvent générer une molécule chromogène.

A cet effet, la présente invention met en oeuvre un substrat chromogène permettant de détecter la présence de l'activité enzymatique d'au moins une enzyme, caractérisé par le fait qu'il est constitué d'au moins deux molécules, une première molécule constituée d'une partie marqueur non chromogène associée à au moins une partie spécifique cible de l'enzyme et une seconde molécule constituée d'une autre substance non chromogène, et que la partie marqueur non chromogène, une fois libérée, réagit avec la seconde molécule pour former une molécule chromogène.

Selon l'invention, la partie marqueur non chromogène de la première molécule est constituée d'aminobenzène ou l'un de ses dérivés: 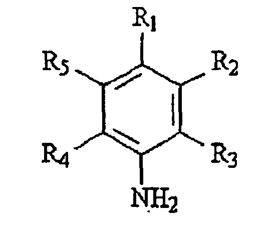 la partie marqueur non chromogène de la seconde molécule est constituée d'α-naphtol ou l'un de ses dérivés : 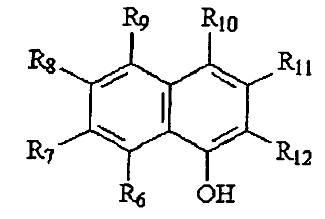 et la molécule chromogène obtenue est constituée par :

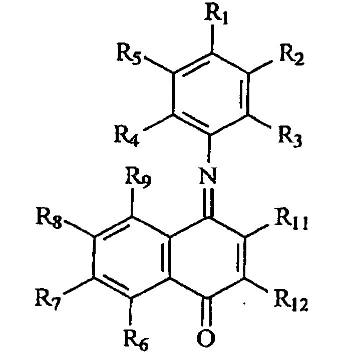

Formules dans lesquelles: le radical R1 est constitué de -OH ou -SH ou 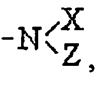 le radical R₁₀ est choisi parmi -H, -Br, -Cl, -I, ou un groupe d'atomes, tel que -SH, qui peut être enlevé durant le couplage oxydatif, et chaque radical R₂ à R₉, R₁₁ ou R₁₂ est choisi parmi -H, -OH, -Br, -Cl, -I, -CH₃, -CH₂CH₃, -OCH₃, -OCH₂CH₃, -COOH.

Dans le cas du radical R₁, X et/ou Z est choisi parmi -H, - CH₃, - CH₂CH₃,

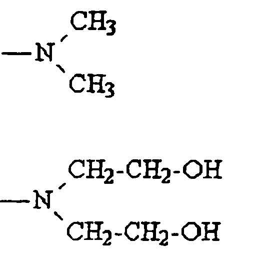

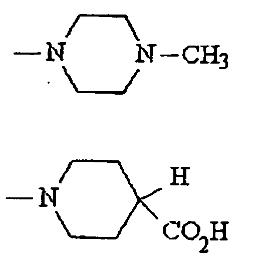

Selon la présente invention, le procédé de détection d'une activité enzymatique par l'intermédiaire de substrats, tels que décrits ci-dessus, consiste à :
- mettre au moins un substrat décrit ci-dessus en présence d'au moins un type de micro-organismes tels que des bactéries, contenu dans un échantillon à tester, sans l'addition d'oxydase,
- attendre que le(s) bactéries hydrolysent le(s) substrat(s), et
- détecter la formation d'un complexe coloré à partir du couplage oxydatif des deux parties marqueur.

Un autre objet de la présente invention est de proposer un procédé, dans lequel les molécules sont présentes dans un matériau absorbant et sont mis en contact avec l'échantillon, et, après détection, les micro-organismes ainsi prélevés sont remis en suspension afin de permettre des analyses ultérieures (identifications, antibiogrammes, etc.).

Les molécules et autres composés entrant dans la composition réactionnelle contenue dans le matériau absorbant sont :
- la première molécule constituée d'amino-benzène ou un de ses dérivés associé à au moins une partie specifique cible de l'enzyme, et
- la seconde molécule constituée d'α-naphtol ou un de ses dérivés.

La composition réactionnelle contient également un oxydant, tel que le ferrycyanate de potassium.

Parmi les autres composés, il est possible d'avoir également un activateur de la réaction, tel qu'une faible quantité de la première molécule et/ou de la deuxième molécule, présente(s) dans l'échantillon à tester.

Toujours parmi les autres composés, il est possible, que la composition comprenne un liant ou colle, tel que la Poly Vinyl Pyrolidone (PVP).

Le procédé peut être utilisé pour identifier le Gram d'une espèce bactérienne à tester, dans ce cas, la première molécule est constituée par de l'AlaDMpPD, et que la seconde molécule est constituée par de l'α-naphtol.

De plus, la composition du mélange réactionnel absorbé par le matériau absorbant est la suivante :
- α-naphtol de 0,01 à 5 g/l, préférentiellement de 0,1 à 1 g/l, et par exemple de 0,5 g/l.
- ferrycyanate de potassium de 0,01 à 5 g/l, préférentiellement de 0,1 à 1 g/l, et par exemple de 0,5 g/l, et
- AlaDMpPD de 0,01 à 5 g/l, préférentiellement de 0,1 à 1 g/l, et par exemple de 0,35 g/l.

Selon une variante, l'activateur est constitué par de l'Ala-DMpPD à une concentration de 0,01 à 0,5 g/l, préférentiellement de 0,05 à 0,1 g/l, et par exemple de 0.075 g/l.

Selon une autre variante, la composition comprend en outre de 1 à 50 g/l, préférentiellement de 10 à 25 g/l, et par exemple de 15 g/l de PVP.

Un dispositif d'identifcation permettant la mise en oeuvre du procédé d'identification décrit ci-dessus, est constitué par un support, par exemple en plastique, qui est inerte vis-à-vis du matériau absorbant et du ou des substrats qu'il contient et/ou vis-à-vis de l'échantillon testé, sur lequel est monté une tête en matériau absorbant, tel que du viscose.

### I - Nouveaux substrats chromogènes et procédé d'utilisation :

L'invention, qui sera décrite ci-après peut être utilisée pour la détection de tout genre ou type de micro-organismes.

Le substrat chromogène, permettant de détecter la présence de l'activité enzymatique d'au moins une enzyme, est constitué d'au moins deux molécules, une première molécule constituée d'une partie marqueur non chromogène associée à au moins une partie spécifique cible de l'enzyme et une seconde molécule constituée d'une autre substance non chromogène. La partie marqueur non chromogène, une fois libérée, réagit avec la seconde molécule pour former une molécule chromogène.

Dans le cas d'une révélation d'une seule activité enzymatique, il y a deux molécules non chromogènes, c'est-à-dire une première molécule ayant une partie spécifique de l'activité enzymatique à révéler et une partie marqueur alors que la seconde molécule n'est constituée que de la partie marqueur. Les deux parties marqueur sont respectivement constituées par les composés I et II ci-dessous. 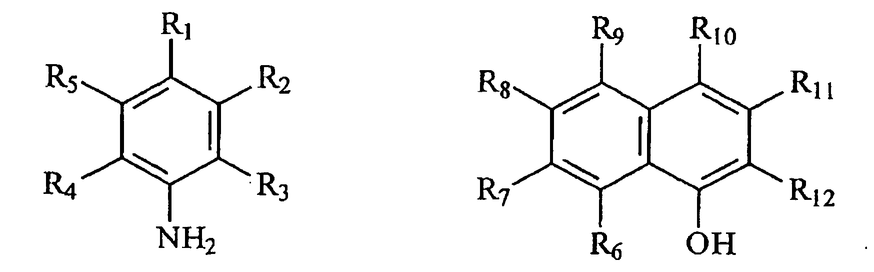 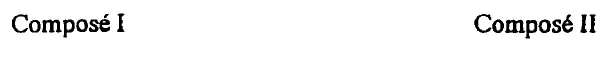 où:
- R1 = -OH ou -SH ou 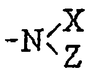 avec X et/nu Z = -H ou autres substituants plus complexes, -CH₃, -CH₂CH₃, 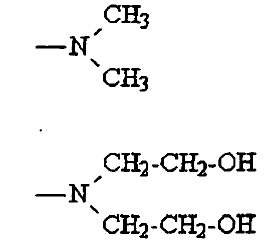 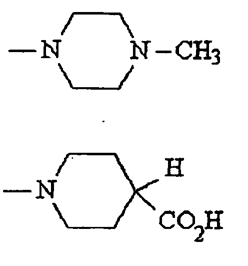
- R₁₀ = -11 ou un groupe ou atome qui peut être enlevé durant le couplage oxydatif, comme -Br, -Cl, -I, -SII, etc.
- R₂, R₃, R₄, R₅, R₇, R₈, R₉, R₁₁ et R₁₂ = -H, -OH, -Br, -Cl, -I, -CH3, - CH2CH3, -OCH3, -OCH2CH3, -COOH,

Le composé III obtenu par la combinaison des deux composés T et Il est donc le suivant :

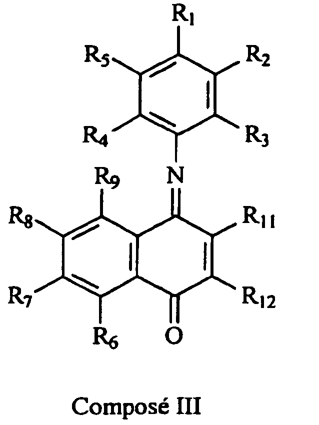

Pour comprendre l'invention, il est rappelé que la formule d'un substrat enzymatique d'hydrolase peut être schématisée sous forme :
- A - O - B, s'il s'agit d'un substrat d'osidase, de phosphatase, ou d'estérase ou de sulfatase.
   L'hydrolyse enzymatique peut alors s'écrire :

   A - O -B + H₂O ⇒ A-OH + B-OH,

   avec selon l'invention :
   A-OH = ose, phosphate, sulfate, acide gras (depuis le plus simple c'est-à-dire l'acide acétique), et
   B-OH = composé I avec R₁ = -OH ou composé II.
- A-CO-NH-B, s'il s'agit d'un substrat de peptidase.
   L'hydrolyse enzymatique peut alors s'écrire :

   A-CO-NII-B + H₂O ⇒ A-COOH+B-NH₂,

   avec selon l'invention :

   A-COOH = acide aminé ou chaîne d'acides aminés terminés ou non

   par un agent bloquant, et

   B-NH₂ - composé I.

Le procédé selon l'invention utilise, d'une part, un substrat enzymatique tel que décrit ci-dessus, à base d'un composé 1 dérivé de l'aminobenzène, avec R₁ = -OH d'autre part, un agent de révélation présent initialement dans le milieu réactionnel, ou pouvant être ajouté pendant ou après la réaction enzymatique, cet agent étant :
le composé II pour la mise en évidence du composé 1 résultant de l'hydrolyse enzymatique des substrats à base de ce dernier composé,

La formation dans le milieu réactionnel du complexe coloré III (dérivé de l'indophénol) à partir du couplage oxydatif de 1 et 11 permet de déceler l'hydrolyse du substrat et donc l'activité enzymatique impliquée.

La couleur du composé III dépend des substituants sur les composés 1 et 11.

Elle est par exemple pourpre, si R₁ = -OH et R₂ à R₁₁ = -H, ou bleue, dans le cas où R₁ = -OH, R₂ et R₅ = -Cl et R₃ à R₁₁ = -H.

Le procédé ainsi décrit est en particulier extrêmement intéressant pour déceler colorimétriquement des activités de type peptidases. En effet, selon l'état de la technique antérieur, le choix des réactions colorées se limitait à l'utilisation de substrats à base de nitroaniline, peu chromogène, et de substrats à base de naphtylamine très toxique, nécessitant l'addition d'un réactif en fin de réaction (sel de diazonium, par exemple) pour déceler l'aminé libérée.

L'invention met en oeuvre un nouveau type de substrats de peptidases, à base d'aminobenzène ou dérivés (composé 1).

Ce dernier, au fur et à mesure qu'il est libéré dans le milieu réactionnel lors de l'hydrolyse enzymatique, forme un complexe fortement coloré (rouge à bleu, généralement) par couplage oxydatif avec l'α-naphtol ou un dérivé (composé II) présent dans ce milieu.

La réaction de couplage peut également être obtenue en fin de test si l'addition du composé II intervient au terme de la réaction enzymatique.

Une autre application des substrats décrits n'appartenant pas à l'invention consiste à associer deux substrats dans le milieu réactionnel, selon le procède, l'un à base du composé 1 et l'autre à base du composé II.

On peut imaginer ainsi tous les types de combinaisons de détection simultanée de deux activités enzymatiques (par exemple, une peptidase et une osidase, une estérase et une osidase, deux osidases, etc). Dans ces cas, il y aura la formation du complexe coloré III uniquement si les deux substrats sont hydrolyses. Cette combinaison peut être très utile pour les tests de diagnostic qui demandent une grande spécificité, notamment lorsqu'il s'agit de caractériser des micro-organismes.

L'un des exemples cités illustre l'intérêt de cette détection simultanée de deux activités enzymatiques dans le domaine de l'identification des bactéries.

Il est aussi possible de donner une double fonctionnalité au composé I. Il est en effet possible de greffer chimiquement sur le composé I, d'une part sur le groupement -NH₂, un acide aminé ou une chaîne d'acides aminés terminés ou non par un agent bloquant, et d'autre part sur R₁, s'il s'agit d'un groupement hydroxyl (-OH), un ose, un phosphate ou un acide gras. On obtient une molécule que l'on peut qualifier de double substrat qui, pour générer le composé I à l'état libre dans le milieu réactionnel doit être hydrolysé par deux activités enzymatiques distinctes, d'une part, une peptidase et, d'autre part, une osidase, phosphatase ou estérase, selon la nature du produit greffé sur R₁.

Ainsi, en présence du composé II, le complexe coloré III ne peut être obtenu que si les deux activités enzymatiques sont présentes. L'intérêt de tels substrats à double fonctionnalité réside dans leur très grande spécificité qui peut être mise à profit pour caractériser des micro-organismes, par exemple.

Dans le même sens, on peut concevoir un mélange d'un substrat à double fonctionnalité à base du composé I, tel que décrit ci-dessus, et un substrat à base du composé II pour la recherche d'une autre osidase, phosphatase ou estérase. Dans ce cas, la production du complexe coloré III nécessite la présence simultanée de trois activités enzymatiques, afin de libérer les composés I et II. Deux activités enzymatiques permettent d'hydrolyser la molécule à base du compose 1 et une activité enzymatique permet d'hydrolyser la molécule à base du composé II. On augmente ainsi encore la spécificité du test.

Selon l'invention, il est aussi possible de combiner différents types de substrats dans le même milieu réactionnel, en particulier des substrats connus dans l'art antérieur et des substrats selon le procédé revendiqué. Cela permet de mettre en oeuvre différentes réactions colorées, et donc de révéler en même temps plusieurs activités enzymatiques au sein du même milieu réactionnel.

L'invention peut s'appliquer à la recherche d'activités enzymatiques dans différents types d'échantillons, biologiques ou non.

Elle peut également être utilisée pour caractériser des micro-organismes comme des bactéries ou des levures. Les tests enzymatiques peuvent alors être pratiqués en milieu liquide dans des tubes individuels ou dans des supports compartimentes, comme les plaques de microtitration, par exemple. Ils peuvent être pratiqués également en milieu gélosé (dans des boîtes de Pétri, par exemple) avec coloration des colonies produisant les activités enzymatiques recherchées.

La présente invention concerne donc un procédé de détection d'une activité enzymatique par l'intermédiaire de substrats, tels que décrits ci-dessus, qui consiste à :
- mettre les substrats en présence d'au moins un type de bactéries,
- attendre que les bactéries hydrolysent les substrats, et
- détecter les activités enzymatiques basée sur la formation d'un complexe coloré à partir du couplage oxydatif des deux parties marqueur.

Le couplage oxydatif est facilité soit par :
- l'ajour d'au moins un oxydant dans le milieu réactionnel,
- la production, lors d'un processus métabolique au sein de ce milieu réactionnel, d'au moins un oxydant,
- la présence d'oxygène endogène dans ledit milieu réactionnel.

Les exemples cités ci-dessous permettront de mieux comprendre l'invention. Ils concernent uniquement le domaine de la bactériologie, mais il est clair que le procédé peut s'appliquer à tous les autres domaines de l'enzymologie, impliquant la recherche d'hydrolases.

### ❖ Exemple 1 : Détection de la β-glucosidase en milieu liquide (méthode 1) : (hors invention)

| Le milieu réactionnel proposé dans cet exemple a pour formulation : | |
|---|---|
| • peptone de viande | 10 g |
| • NaCl | 5 g |
| • α-naphtyl-β -D-glucopyranoside | 0.5 g |
| • 4-aminophénol | 50 mg |
| • H₂O | 1000 ml |
| • pH | 7.0 |

Ce milieu est stérilisé par filtration sur filtre Millipore 0,22 µ et réparti dans des tubes stérilet, à raison de 5 ml par tube.

Le test de recherche de la β-glucosidasc est pratiqué sur 4 souches cultivées pendant 24H à 35-37°C sur gélose Trypticase-Soja. Ces souches appartiennent' respectivement aux espèces :
- *Escherichia coli,*
- *Staphylococcus sciuri,*
- *Streptococcus pyogenes,*
- *Enterococcus faecalis*

A partir des cultures précédentes, on réalise des suspensions aqueuses ajustées à 0,5 McFarland.
100 microlitres de ces suspensions sont utilisés pour ensemencer quatre tubes de milieu réactionnel, chaque tube correspondant à une souche.
Les tubes bouchés sont incubés à 35-37°C pendant 18 à 24H.

Après l'incubation, on observe l'apparition d'une coloration pourpre correspondant à la formation du complexe indophénol et traduisant la présence d'une activité β-glucosidase.

Les résultats obtenus sont les suivants :

| **souche** | **résultat théorique attendu** | **résultat obtenu** |
|---|---|---|
| *E. coli* | - | - |
| *S. sciuri* | + | + |
| *S. pyogenes* | - | - |
| *E. faec*alis | 1 | + |

### ❖ Exemple 2 : Détection de la β-glucosidase en milieu liquide (méthode 2) : (hors invention)

| Le milieu réactionnel proposé dans cet exemple a pour formulation: | |
|---|---|
| • peptone de viande | 10 g |
| • NaCl | 5 g |
| • α-naphtol | 50 mg |
| • 4-minophényl-β-D-glucopyranoside | 0.5 g |
| • H₂O | 1000 ml |
| • pII | 7.0 |

La préparation du milieu, le choix des souches testées et la pratique du test sont identiques la procédure décrite dans l'exemple 1.
L'apparition d'une coloration pourpre due à la formation du complexe indophénol indique la présence d'une activité β-glucosidase.

Les résultats obtenus sont identiques à ceux de l'exemple 1, à savoir :

| **souche** | **résultat théorique attendu** | **résultat obtenu** |
|---|---|---|
| *E. coli* | - | - |
| *S. sciuri* | l | + |
| *S. pyogenes* | - | - |
| *E. faecalis* | + | + |

Ces résultats montrent qu'il est possible de détecter une activité de typé osidase en utilisant selon l'invention aussi bien un substrat à base du composé I qu'un substrat à base du composé II. Il en serait de même pour rechercher des activités de types estérases ou phosphatases.

### ❖ Exemple 3 - Détection de la pyroglutamyl-aminopeptidase en milieu liquide:

| Le milieu réactionnel propose dans cet exemple a pour formulation : | |
|---|---|
| • peptone de viande | 10 g |
| • NaCl | 5 g |
| • α-naphtol | 50 mg |
| • L-pyroglutamyl-4-amino-2,6-dichlorophénol | 0.5 g |
| • H₂O | 1000 ml |
| • pH | 7.0 |

La préparation du milieu, le choix des souches testées et la pratique du test sont identiques à la procédure décrite dans l'exemple 1.

L'apparition d'une coloration bleue due à la formation du complexe indophénol met en évidence une activité pyroglutamyl-aminopeptidase. A noter que la teinte bleue résulte de la présence de groupements chlore en position 2 et 6 sur le 4-aminophénol.

Les résultats obtenus sont les suivants :

| **souche** | **résultat théorique attendu** | **résultat obtenu** |
|---|---|---|
| *E. coli* | - | - |
| *S. sciuri* | - | - |
| *S. pyogenes* | + | + |
| *E. faecalis* | + | + |

### ❖ Exemple 4 : Recherche combinée de deux activités enzymatiques selon le procédé de l'invention. Application à la détection simultanée de la β-glucosidase et de la pyroglutamyl-aminopeptidase: (hors invention)

| Le milieu réactionnel proposé dans cet exemple a pour formulation : | |
|---|---|
| • peptone de viande | 10 g |
| • NaCl | 5 g |
| • α-naphtyl-b-D-glucopyranoside | 0.5 g |
| • L-pyroglutamyl-4-amino-2,6-dichlorophénol | 0.5 g |
| • H₂O | 1000 ml |
| • pH | 7.0 |

La préparation du milieu, le choix des souches testées et la pratique du test sont identiques à la procédure décrite dans l'exemple 1.
L'apparition d'une coloration bleue due à la formation du complexe indophénol n'est possible que si les deux substrats sont hydrolysés. Elle traduit donc une combinaison d'activités β-glucosidase et pyroglutamyl-aminopeptidase dans le même milieu réactionnel. L'absence de l'une des activités empêche la formation du complexe coloré.

Les résultats obtenus sont les suivants :

| **souche** | **resultat théorique attendu** | **résultat obtenu** |
|---|---|---|
| *E. coli* | - | - |
| *S. sciuri* | - | - |
| *S. pyogenes* | - | - |
| *E. faecalis* | + | + |

Cet exemple illustre clairement un des avantages de l'invention en permettant une plus grande sélectivité dans la caractérisation des microorganismes (*E*. *faecalis,* dans certe application).

### ❖ Exemple 5 : Caractérisation de bactéries en milieu gélosé par recherche simultanée des activités β-galactosidase (βGAL) et L-alanine-aminopeptidase (Ala) : (hors invention)

A un milieu trypticase soja gélosé, sont ajoutés de l'α-naphtyl-β-D-galactopyranoside et de la L-alanyl-N,N'-diméthyl-p-phénylène-diamine-naphtalène sulfonylhydrazide. Ce substrat est dérivé du composé 1 selon l'invention.

Le milieu est réparti et ensemence de la façon suivante :
- six boîtes avec des cultures pures:
   - *Escherichia coli* | βGAL (+), Ala (+)]
   - *Klebsiella pneumoniae* [βGAL (+), Ala (+)]
   - *Salmonella typhimurium* [βGAL (-), Ala (+)]
   - *Pseudomonas aeruginosa* [βGAL (-), Ala (+)]
   - *Staphylococcus xylosus* [βGAL (+), Ala (-)]
   - *Candida albicans* [βGAl. (-), Ala (-)].
- deux boîtes avec les mélanges de souches *E*. *coli* / *S. xylosus* et *S*. *typhimurium* / *C. albicans.*

Les milieux sont incubés à 35-37°C. Après 18 - 24H d'incubation, seules les boîtes contenant les souches d'*E*. *coli* et *K. pneumoniae* [βGAL (+), Ala (+)] présentent des colonies colorées en bleu.

Avec le mélange *E. coli* / *S*. *xylosus,* seules les colonies d'*E*. *coli* sont bleues, celles de *S*. *xylosus* restant incolores.

Tout comme dans l'exemple 4, la production du complexe coloré III à base d'indophénol a donc nécessité la présence conjointe de deux activités enzymatiques, ici la β-galactosidase et la L-alanine-aminopeptidase.

En revanche, la présence de l'une seulement ou aucune de ces deux activités n'entraîne pas la formation du complexe coloré.

Comme cela est le cas dans l'exemple 5, le choix d'un dérivé approprié de la phénylène-diamine permet d'obtenir une coloration qui reste au voisinage immédiat des colonies et ainsi de distinguer deux populations différentes en mélange.

Cet exemple montre qu'il est possible, selon l'invention, de coloré spécifiquement des colonies la surface d'un milieu gélosé et donc de les caractériser. Dans le cas présent, il pourrait s'appliquer à la détection des bactéries conformes qui sont des bacilles à Gram négatif possédant les deux activités β-galactosidase et L-alanine-itminopeptidase.

### ❖Exemple 6 : Recherche combinée de trois activités enzymatiques selon le procédé de l'invention. Application à la détection simultanée de la β-galactosidase (βGAL), de la β-glucosidase (βGLU) et de la L-alanine-aminopeptidase (Ala): (hors invention)

| Le milieu réactionnel proposé dans cet exemple a pour formulation : | |
|---|---|
| • peptone de viande | 10 g |
| • NaCl | 5 g |
| • α-naphtyl-β-D-glucopyranoside | 0.5 g |
| • L-alanyl-4-amino-2,6-dichlorophényl-β -D-galactopyranoside | 0.5 g |
| • H₂O | 1000 ml |
| •pH | 7.0 |

La préparation du milieu est identique à la procédure décrite dans l'exemple 1.

Le test de recherche des 3 activités enzymatiques est pratiqué sur 5 souches cultivées pendant 241J à 35-37°C sur gélose Trypticase-Soja. Ces souches appartiennent respectivement aux espèces:
- *Escherichia coli* [βGAL (+),βGLU (-), Ala (+)]
   - *Klebsiella pneumoniae* [βGAI, (+),βGLU (+), Ala (+)]
   - *Salmonella typhimurium* [βGAL (-),βGLU (-), Ala (+ )]
   - *Stenotrophomonas maltophilia* [βGAL (-),βGLU (+), Ala (+)]
   - *Staphylococcus saprophyticus* [βGAL (+),βGLU (-), Ala (-)]

L'apparition d'une coloration bleue due à la formation du complexe indophénol n'est possible que si les 3 substrats sont hydrolysés. Elle traduit donc une combinaison d'activités β-galactosidase, β-glucosidase et L-alanine-aminopeptidase dans le même milieu réactionnel, comme c'est le cas pour la souche de *K. pneumoniae.* L'absence de l'une des activités empêche la formation du complexe coloré.

Les résultats obtenus sont les suivants :

| **souche** | **résultat théorique attendu** | **résultat obtenu** |
|---|---|---|
| *E. coli* | - | - |
| *K. pneumoniae* | + | + |
| *S. typhimurium* | - | - |
| *S. maltophilia* | - | - |
| *S. sapropriticus* | - | - |

Cet exemple montre une nouvelle fois les potentialités de l'invention pour mettre en oeuvre des tests hautement sélectifs.

Les six exemples cités décrivent uniquement des tests enzymatiques selon l'invention.

Il est bien entendu possible de combiner ceux-ci aux tests connus dans l'art antérieur, utilisant des substrats chromogéniques ou fluorogéniques.

### II - Procédé et dispositif d'identification:

Le principe du milieu, utilisé avec le procédé et le dispositif d'identification, est basé sur l'utilisation des substrats jumelés décrits précédemment.

Selon un exemple représentatif, le procédé d'identification des GRAM utilise un substrat à base d'amino-benzène ou un de ses dérivés, tel que le DiMéthyl-paraPhénylène-Diamine (DMpPD). Dans l'exemple qui suit, le substrat est constitué par de l'Alanine-Diméthyl-paraPhénylène-Diamine (AlaDMpPD). L'activité mise en évidence est donc l'Alanine-aminopeptidase, activité spécifique des bactéries à Gram négatif.

Son fonctionnement est le suivant. Il y a, par hydrolyse enzymatique, libération du groupement DMpPD. Ensuite le DMpPD se lie avec l'α-naphtol (ou un dérivé) par couplage oxydatif facilité par un oxydant, le ferrycyanate de potassium (K₁Fc(CN)₆). Il y a alors formation d'un complexe coloré gris violet.

Dans un premier temps, le dispositif est défini de la manière suivante :
- un milieu biogélytone contenant comme substrat : l'AlaDMpPD, et
- un écouvillon imprégné d'un mélange réactionnel constitué d'α-naphtol et de ferrycyanate de potassium.

Un autre dispositif a été défini qui est encore plus spécifique dans l'identification du Gram et est également utilisable avec les géloses au sang de type Columbia. Il consiste en :
- un milieu constitué d'une gélose au sang de type Columbia (avec ou sans AlaDMpPD), et
- un mélange réactionnel qui imprègne un écouvillon et qui est constitué à base d'α-naphtol, de ferrycyanate de potassium et d'AlaDMpPD puis séché.

Ce mélange réactionnel contenu par l'écouvillon, a été encore amélioré par l'ajout d'une étape de séchage des écouvillons, ce qui facilite leur manipulation ultérieure.

La composition exacte est la suivante :
- pour la gélose: présence ou non d'AlaDMpPD à 0, 075 gramme par litre (g/l), et
- pour l'écouvillon :
   - α-naphtol de 0,01 à 5g/l, préférentiellement de 0,1 à 1g/l, et par exemple de 0,5g/l,
   - ferrycyanate de potassium de; 0,0 1 à 5 g/l, préférentiellement de 0,1 à 1g/l, et par exemple de 0,5 g/1, et
   - AlaDMpPD de 0,01 à 5g/l, préférentiellement de 0,1 à 1g/l, et par exemple de 0,35 g/l.

Les résultats obtenus en terme de sensibilité et spécificité sont alors les meilleurs obtenus. Dans ce cas, la détection de la réaction n'est pas instantanée. Il faut attendre 15 à 30 secondes (s) pour observer l'apparition d'une éventuelle coloration, temps nécessaire à l'hydrolyse du substrat par l'aminopeptidase bactérienne.

Soixante et une souches sur différents milieux tels que : gélose chocolat Polyvitex (marque déposée), milieu CPS ID2 (marque déposée), TSA +/- sang et Columbia +/- sang, ont été testées avec des écouvillons imprégnés d'α-naphtol, de ferrycyanate de potassium et d'AlaDMpPD puis séchés. Cette étude a montré que ce système était compatible avec la majorité des milieux conventionnels et chromogéniques usuels.

Pour améliorer encore le temps de réaction, un activateur de la réaction est incorporé dans le milieu.

Dans ce contexte d'activation de la réaction, il s'est avéré que l'ajout dans le milieu d'une faible quantité d'AlaDMpPD et/ou d'α-naphtol permet la détection de la réaction sans diminuer la sensibilité. De plus, le temps de détection est ainsi considérablement diminué, puisqu'il est porté de 0 à 10 s. Cette faible quantité correspond à une concentration de 0,01 à 0,5 g/l, préférentiellement de 0,05 à 0,1 g/l, et par exemple de 0,075 g/l.

Scion une variante de réalisation, il est également possible d'ajouter d'un liant sur l'écouvillon. L'apport d'un liant permet à la fois de resserrer les fibres de la tête de l'écouvillon et de limiter le relargage de produits présents sur l'écouvillon dans la suspension de l'inoculum. Un tel liant peut être constitué par la PolyVinyl Pyrolidone (PVP), qui permet en effet non seulement de répondre aux critères précédents mais aussi d'augmenter sensiblement la spécificité. Dans la composition précédemment décrite avec les concentrations, le PVP est introduit dans les constituants de la tête viscose de l'écouvillon dans une concentration de 1 a 50 g/l, préférentiellement de 10 à 25 g/l, et par exemple de 15 g/l.

### 1°) Expérimentation :

Une évaluation de ce milieu a donc été réalisée sur cent trente trois (133) souches reparties selon les espèces suivantes. Le tableau 1, qui suit, énumère les espèces testées ainsi que le nombre de souche pour chaque espèce.

**Tableau 1: Espèces testées et nombre de souches pour chaque espèce**

| **Espèce** | **Nombre de souches** |
|---|---|
| *Acinetobacter baumanii* | 2 |
| *Acinetobacter junii* | 2 |
| *Citrobacter freundii* | 3 |
| *Citrobacter spp* | 1 |
| *Enterobacter cloacae* | 3 |
| *Enterobacter intermidis* | 1 |
| *Enterobacter spp* | 1 |
| *Escherichia coli* | 4 |
| *Klebsiella oxytoca* | 5 |
| *Klebsiella pneumoniae* | 4 |
| *Morganella morganii* | 4 |
| *Proteus mirabilis* | 5 |
| *Proteus vulgaris* | 5 |
| *Providencia stuartii* | 4 |
| *Pseudomonas aeruginosa* | 4 |
| *Pseudomonas fluorescens* | 5 |
| *Serratia marcescens* | 4 |
| *Bacillus cereus* | 4 |
| *Bacillus lentus* | 1 |
| *Bacillus mycoïdes* | 2 |
| *Bacillus subtilis* | 2 |
| *Corynebacterium aquaticum* | 2 |
| *Corynebacterium ulcerans* | 2 |
| *Enterococcus faecalis* | 5 |
| *Enterococcus faccium* | 3 |
| *Enterococcus gallinarum* | 3 |
| *Haemophilus haemolyticus* | 2 |
| *Haemophilus influenzae* | 2 |
| *Lactobacillus casei* | 4 |
| *Listeria monocytogenes* | 4 |
| *Staphylococcus aureus* | 3 |
| *Staphylococcus epidermidis* | 3 |
| *Staphylococcus haemolyticus* | 3 |
| *Staphylococcus saprophyticus* | 3 |
| *Streptococcus agalactiae* | 4 |
| *Streptococcus pneumoniae* | 3 |
| *Streptococcus pyogenes* | 3 |
| *Candida albicans* | 3 |
| *Candida glabrata* | 3 |
| *Candida guilliermondii* | 1 |
| *Candida kefyr* | 1 |
| *Candida krusei* | 3 |
| *Candida parapsilosis* | 1 |
| *Candida tropicalis* | 4 |
| *Trichosporon asteroïdes* | 1 |
| *Trichosporon mucoides* | 1 |
| TOTAL | 133 |

Le tableau 2 énumère les résultats obtenus :

**Tableau 2 : Sensibilité et spécificité des espèces testées**

| | |
|---|---|
| Sensibilité | 93 % |
| Spécificité vis-à-vis des Gram + | 81 à 90 % |
| Spécificité vis-à-vis des levures | 89 à 100 % |

Parmi les résultats obtenus sur les souches testées, certaines souches sont des faux négatifs. Ces faux négatifs sont deux (2) des quatre (4) souches de *P. aeruginosa,* deux (2) des cinq (5) souches de *P. fluorescens,* et une (1) des deux (2) souches *d'H*. *influenzae,* Certaines souches sont des faux positifs. Ces faux positifs sont constitués par les deux (2) souches de *C*. *ulcerans,* une (1) des cinq (5) souches d*'E. faecalis,* les trois (3) souches de *S*. *pyogenes,* deux (2) des quatre (4) souches de *S*. *agalactiae,* la souche de *C*. *guilliermondii* et la souche de *C*. *parapsilosis.*

### 2°) Lisibilité du test:

La quasi-totalité des bactéries à Gram négatif, à l'exception des *Pseudomonas,* donnent une coloration gris violette franche dont l'intensité est supérieure strictement à 2 (sur une échelle semi-quantitative allant jusqu'à 4), tandis que les bactéries à Gram positif ou qui sont faux-positifs, à l'exception de *S. pyogenes,* ont une intensité de coloration inférieure à 1 sur cette même échelle.

### 3°) Temps de détection:

Environ 90% des bactéries à Gram négatif sont détectées entre 0 et 10 s. la plupart des bactéries à Gram positif ou qui sont faux-positifs, sont détectées après 10 s, à l'exception des souches de *S*. *pyogenes.*

Le milieu actuel présente donc une bonne sensibilité et une bonne spécificité. Les autres souches faux-positifs sont généralement d'intensité très faible et présentent un temps de détection supérieur à 10 s. Ces souches sont donc plutôt des souches douteuses plutôt que des faux-positifs.

### 4°) Définition de l'écouvillon:

Le modèle d'écouvillon, particulièrement intéressant, est un écouvillon qui répond aux critères suivants :
- un bâtonnet plastique de diamètre 2,5 millimètres (mm) et de longueur 150 mm, et
- avec une tête viscose de diamètre inférieur ou égal à 4,5 mm.

### 5") Mode de fabrication et de manipulation des écouvillons :

Les conditions de fabrication sont les suivantes :
- inhibition dans une solution telle que définie ci-dessus pendant 2 à 3 minutes (mn),
- séchage pendant 2 heures (h) à 37°C, et
- conditionnement en sac plastique étanche et à l'abri de la lumière.

### 6°) Conditionnement des écouvillons :

Les écouvillons peuvent par exemple être stockés de manière stérile (rayons gamma) dans un emballage contenant un (1), vingt-cinq (25) ou cent (100) à mille (1000) de ces écouvillons. Néanmoins, un conditionnement de vingt (20) à vingt-cinq (25) écouvillons pas obligatoirement stérilisés après séchage est tout à fait envisageable. Le conditionnement s'effectue avec ou sans substance de dessiccation, et à l'abri de la lumière.

### 7°) Paramètres critiques :

L'utilisation des substrats jumelés est donc identique à celle qui est exposée dans le début de la description. Ainsi, la tête de l'écouvillon contient au moins deux molécules. Ainsi dans l'exemple donné, une première molécule (AlaDMpPD) est constituée d'une partie marqueur (DMpPD) non chromogène associée à au moins une partie spécifique (alanine) cible de l'enzyme (Alanine-aminopeptidase) et une seconde molécule constituée d'une autre substance non chromogène (α-naphtol), la partie marqueur non chromogène (AlaDMpPD), une fois libérée, réagit avec la seconde molécule (α-naphtol) pour former une molécule chromogène.

L'utilisation de l'écouvillon sert de support de réactifs et permet la réalisation de la suspension de l'inoculum, qui sera utilisé dans d'autres systèmes d'identification et antibiogramme, sur la hase des mêmes souches qui ont été évaluées précédemment. Ceci diminue donc considérablement les erreurs lors de repiquage de nouveaux micro-organismes « semblables » pour effectuer les opérations suivantes d'identification et d'antibiogramme, etc.

De plus, il est également possible de réaliser des écouvillons pour d'autres types de tests, par exemple oxydase, indole, estérase, phosphatase...

## Revendications

1. Procédé de détection d'une activité enzymatique peptidase, **caractérisé en ce qu'**il consiste à :
- mettre au moins un substrat constitué d'au moins deux molécules,
• une première molécule constituée d'une partie marqueur non chromogène associée à au moins une partie spécifique cible de l'enzyme, ladite partie marqueur non chromogène étant constituée d'aminobenzène ou de l'un de ses dérivés, , 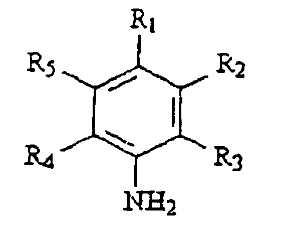
• et une seconde molécule constituée d'une partie marqueur non chromogène constituée d'α-naphtol ou de l'un de ses dérivés, 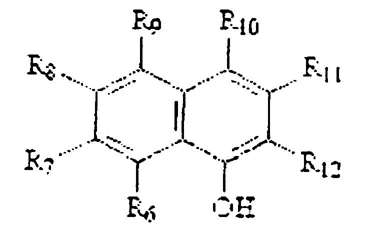 en présence d'au moins un type de micro-organismes tels que des bactéries contenu dans un échantillon à tester, sans l'addition d'oxydase
- attendre que les bactéries hydrolysent le(s) substrat(s), et
- détecter la formation d'un complexe coloré à partir du couplage oxydatif des deux parties marqueur, ledit complexe coloré correspondant à la molécule 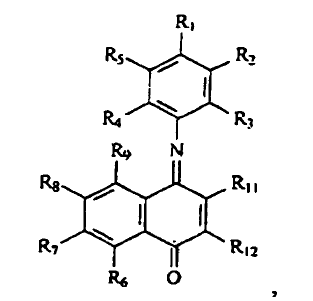 formules dans lesquelles :
o le radical R1 est constitué de OH, SH ou N(X)(Z) où X et/ou Z sont choisis parmi H, CH₃, CH₂CH₃, N(CH₃)₂, N(CH₂CH₂OH)₂, et 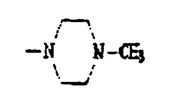 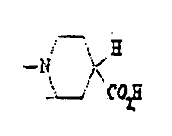
o le radical R10 est choisi parmi -H, Br, Cl, et I, et les groupes d'atomes tels que SH, qui peut être enlevé durant le couplage oxydatif
o et chaque radical R2 à R9, R11 et R12 est choisi parmi H, OH, Br, Cl, et I, CH₃, CH₂CH₃, OCH₃, OCH₂CH₃ et COOH.

2. Procédé, selon la revendication 1, **caractérisé en ce que** lesdites molécules sont présentes dans un matériau absorbant et sont mises en contact avec l'échantillon, et **en ce que**, après détection, les micro-organismes ainsi prélevés sont remis en suspension afin de permettre des analyses ultérieures (identifications, antibiogrammes, etc.).

3. Procédé, selon la revendication 2, **caractérisé en ce que** lesdites molécules sont associées à un oxydant.

4. Procédé selon la revendication 3, **caractérisé en ce que** l'oxydant est le ferrycyanate de potassium.

5. Procédé, selonles revendications 1 à 4, **caractérisé en ce qu'**il comprend un activateur de la réaction, tel qu'une faible quantité de la première molécule et/ou de la deuxième molécule, présente(s) dans l'échantillon à tester.

6. Procédé, selon l'une des revendications 1 à 5, **caractérisé en ce que** la composition comprend un liant, tel que le Poly Vinyl Pyrolidone (PVP).

7. Procédé, selon l'une des revendications 1 à 6, pour identifier le Gram d'une espèce bactérienne à tester, **caractérisé en ce que** la première molécule est constituée par de l'AlaDMpPD, et que la seconde molécule est constituée par de l'α-naphtol.

8. Procédé, selon l'une quelconque des revendications 1 à 6 en combinaison avec la revendication 7, **caractérisé en ce que** la composition du matériau absorbant est la suivante :
- α-naphtol de 0,01 à 5 g/l, préférentiellement de 0,1 à 1 g/l, et par exemple de 0,5 g/l,
- ferrycyanate de potassium de 0,01 à 5 g/l, préférentiellement de 0,1 à 1 g/l, et par exemple de 0,5 g/l, et
- AlaDMpPD de 0,01 à 5 g/l, préférentiellement de 0,1 à 1 g/l, et par exemple de 0, 5 g/l.

9. Procédé, selon la revendication 5, **caractérisé en ce que** l'activateur est constitué par de l'Ala-DMpPD à une concentration de 0,01 à 0,5 g/l, préférentiellement de 0,05 à 0,1 g/l, et par exemple de 0 ,075 g/l.

10. Procédé, selon la revendication 6, **caractérisé en ce que** la composition comprend en outre de 1 à 5 0 g/l, préférentiellement de 10 à 2 5 g/l, et par exemple de 15 g/l de PVP.

## Claims

1. Process for detecting a peptidase enzymatic activity, **characterized in that** it consists in:
- placing at least one substrate consisting of at least two molecules,
a first molecule consisting of a non-chromogenic labeling portion associated with at least one specific target portion of the enzyme, said non-chromogenic labeling portion consisting of aminobenzene or a derivative thereof: 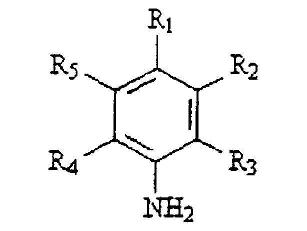 And a second molecule consisting of a non-chromogenic labeling portion consisting of α-naphthol or a derivative thereof: 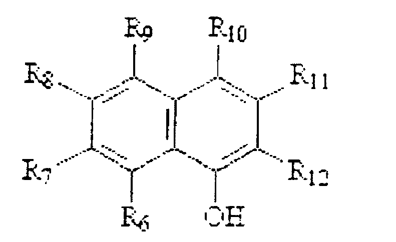 in the presence of at least one type of microorganisms such as bacteria which are contained in a test sample, without adding oxidase,
- waiting for the bacteria to hydrolyse the substrate(s), and
- detecting the formation of a coloured complex from the oxidative coupling of the two labeling portions, said coloured complex consisting of: 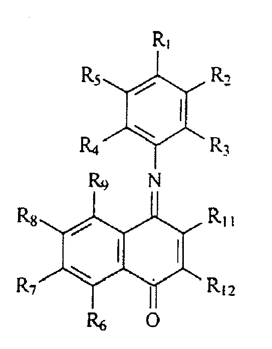 formulae in which:
the radical R₁ consists of OH, SH or N(X)(Z) wherein
X and/or Z are selected from H, CH₃, CH₂CH₃, N(CH₃)₂, N(CH₂CH₂OH)₂ and 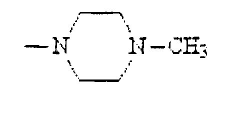 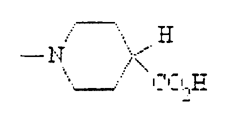
the radical R₁₀ is selected from -H, Br, Cl and I, and the groups of atoms such as SH, that may be removed during the oxidative coupling,
and each radical R₂ to R₉, R₁₁ and R₁₂ is selected from H, OH, Br, Cl, and I, CH₃, CH₂CH₃, OCH₃, OCH₂CH₃ and COOH.

2. Process, according to Claim 1, **characterized in that** said molecules are present in an absorbent material and are placed in contact with the sample, and **in that**, after detection, the microorganisms thus withdrawn are resuspended in order to allow subsequent analyses (identifications, antibiotic assays, etc.).

3. Process, according to claim 2, **characterized in that** said molecules are associated with an oxidizing agent.

4. Process according to claim 3, **characterized in that** the oxidizing agent is potassium ferricyanide.

5. Process, according to claims 1 to 4, **characterized in that** the reaction composition comprises a reaction activator, such as a small amount of the first molecule and/or of the second molecule, which is (are) present in the sample to be tested.

6. Process, according to any one of claims 1 to 5, **characterized in that** the composition comprises a binder, such as PolyVinylPyrrolidone (PVP).

7. Process, according to any one of claims 1 to 6, for the Gram identification of a bacterial species to be tested, **characterized in that** the first molecule consists of AlaDMpPD, and **in that** the second molecule consists of α-naphthol.

8. Process, according to any one of claims 1 to 6 in combination with claim 7, **characterized in that** the composition of the absorbent material is as follows:
- α-naphthol from 0.01 to 5 g/l, preferably from 0.1 to 1 g/l, and for example 0.5 g/l,
- potassium ferricyanide from 0.01 to 5 g/l, preferably from 0.1 to 1 g/l, and for example 0.5 g/l, and
- AlaDMpPD from 0.01 to 5 g/l, preferably from 0.1 to 1 g/l, and for example 0.5 g/l.

9. Process according to claim 5, **characterized in that** the activator consists of AlaDMpPD at a concentration of from 0.01 to 0.5 g/l, preferably from 0.05 to 0.1 g/l, and for example 0.075 g/l.

10. Process, according to Claim 6, **characterized in that** the composition also comprises from 1 to 50 g/l, preferably from 10 to 25 g/l, and for example 15 g/l of PVP.

## Patentansprüche

1. Verfahren zum Nachweisen von einer Enzymaktivität vom Typ Peptidase, **dadurch gekennzeichnet, dass** es umfasst:
- Bringen von mindestens einem Substrate, das aus mindestens zwei Molekülen gebildet ist,
einem ersten Molekül, das aus einem nicht chromogenen Marker-Bestandteil, der mit mindestens einem spezifischen Bestandteil, der ein Angriffsziel für das Enzym darstellt, assoziiert ist, aufgebaut ist, wobei der nicht chromogene Marker-Bestandteil aus Aminobenzol oder einem seiner Derivate gebildet ist: 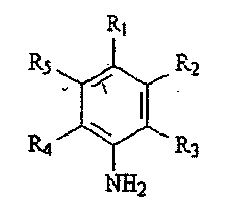 und einem zweiten Molekül, das aus einem nicht chromogenen Marker-Bestandteil, der aus α-Naphthol oder einem seiner Derivate gebildet ist: 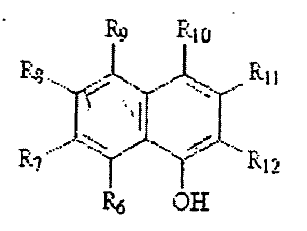 in Gegenwart mindestens einer Mikroorganismen-art wie Bakterien, die in einer zu untersuchenden Probe enthalten ist, ohne Oxydase einzufügen;
- Warten, bis die Bakterien das (die) Substrat(e) hydrolysieren, und
- Nachweisen der Bildung eines Farbkomplexes durch oxidative Kopplung der zwei Marker-Bestandteile, und die Farbkomplexe aus dem Molekül 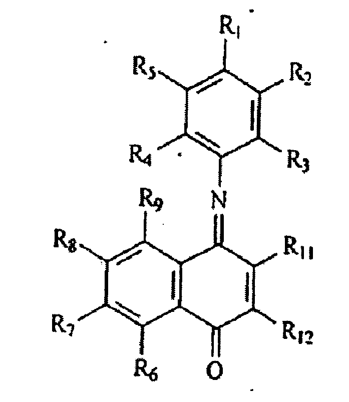 gebildet ist,
wobei in den Formeln:
das Radikal R₁ aus OH, SH oder N (X) (Z) wobei X und/oder
Z aus H, CH₃, CH₂CH₃, N(CH₃)₂, N(CH₂CH₂OH)₂, gebildet ist, und 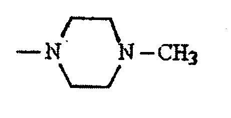 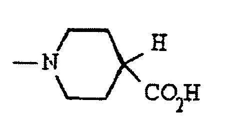
gewählt ist,
das Radikal R₁₀ aus -H, Br, Cl, I und den bei der oxidativen Kopplung abgelösten Atomgruppen wie SH gewählt ist,
jedes Radikal R₂ bis R₉, R₁₁ und R₁₂ aus H, OH, Br, Cl, I, CH₃, CH₂-CH₃, OCH₃, OCH₂CH₃ und COOH gewählt ist

2. Verfahren nach Anspruch 1, **dadurch gekennzeichnet, dass** die Moleküle in einem absorbierenden Stoff vorliegen und mit der Probe in Kontakt gebracht sind, und dass nach dem Nachweis die folglich entnommenen Bakterien wieder in Suspension gebracht werden, um weitere Analysen (Identifizierungen, Antibiogramme usw.) zu ermöglichen.

3. Verfahren nach Anspruch 2, **dadurch gekennzeichnet, dass** die Moleküle mit ein Oxidationsmittel assoziiert sind.

4. Verfahren nach Anspruch 3, **dadurch gekennzeichnet, dass** das Oxidationsmittel Kaliumhexacyanoferrat(III) ist.

5. Verfahren nach Anspruche 1 bis 4, **dadurch gekennzeichnet, dass** die reaktive Zusammensetzung ein Reaktionsaktivierungsmittel umfasst, wie etwa eine geringe Menge des ersten Moleküls und/oder des zweiten Moleküls, die in der zu untersuchenden Probe vorliegt.

6. Verfahren nach einem der Ansprüche 1 bis 5, **dadurch gekennzeichnet, dass** die Zusammensetzung ein Bindemittel wie Polyvinylpyrrolidon (PVP) umfasst.

7. Verfahren nach einem der Ansprüche 1 bis 6, um die Gramfärbung einer Bakterienart, auf die untersucht wird, zu bestimmen, **dadurch gekennzeichnet, dass** das erste Molekül aus AlaDMpPD gebildet ist und das zweite Molekül aus α-Naphthol gebildet ist.

8. Verfahren nach einem der Ansprüche 1 bis 6 in Kombination mit Anspruch 7, **dadurch gekennzeichnet, dass** die Zusammensetzung des absorbierenden Stoffes wie folgt ist:
- 0,01 bis 5 g/l, vorzugsweise 0,1 bis 1 g/l, und beispielsweise 0,5 g/l α-Naphthol,
- 0,01 bis 5 g/l, vorzugsweise 0,1 bis 1 g/l, und beispielsweise 0,5 g/l Kaliumhexacyanoferrat (III),
- 0,01 bis 5 g/l, vorzugsweise 0,1 bis 1 g/l, und beispielsweise 0,5 g/l AlaDMpPD.

9. Verfahren nach Anspruch 5, **dadurch gekennzeichnet, dass** das Aktivierungsmittel aus AlaDMpPD mit einer Konzentration von 0,01 bis 0,5 g/l, vorzugsweise von 0,05 bis 0,1 g/l, und beispielsweise von 0,075 g/l besteht.

10. Verfahren nach Anspruch 6, **dadurch gekennzeichnet, dass** die Zusammensetzung außerdem 1 bis 50 g/l, vorzugsweise 10 bis 25 g/l, und beispielsweise 15 g/l, PVP umfasst.
